(19) Europäisches Patentamt
European Patent Office
Office européen des brevets

(11) Numéro de publication: **0 151 084**
**A2**

(12) # DEMANDE DE BREVET EUROPEEN

(21) Numéro de dépôt: **85420014.4**

(22) Date de dépôt: **23.01.85**

(51) Int. Cl.⁴: **C 07 D 405/06**, A 01 N 43/50, A 01 N 43/653, C 07 D 307/20, C 07 D 317/20, C 07 D 303/22, C 07 C 43/315, C 07 D 407/06

(30) Priorité: **26.01.84 FR 8401424**

(43) Date de publication de la demande: **07.08.85 Bulletin 85/32**

(84) Etats contractants désignés: **AT BE CH DE FR GB IT LI LU NL SE**

(71) Demandeur: **RHONE-POULENC AGROCHIMIE, 14-20, rue Pierre Baizet, F-69009 Lyon (FR)**

(72) Inventeur: **Debourge, Jean-Claude, 14 Avenue des Frères Lumière, F-69410 - Champagne au Mont D'Or (FR)**
Inventeur: **Greiner, Alfred, 2, rue Mouillard, F-69009 - Lyon (FR)**

(74) Mandataire: **Brachotte, Charles et al, RHONE-POULENC AGROCHIMIE P.I.D. BP 9163, F-69263 Lyon Cedex 09 (FR)**

(54) **Fongicides à groupes triazole et oligoéther.**

(57) Fongicides, utilisables notamment contre les maladies des céréales, et ayant pour formule:

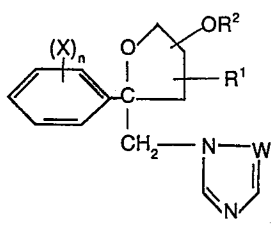

dans laquelle:
X est un atome d'halogène ou un groupe cyano ou nitro, ou un groupe alkyle ou alcoxy éventuellement halogéné,
n est un nombre entier, positif ou nul, inférieur à 6,
W représente un groupe trivalent constitué soit d'un groupe =CH–, soit d'un atome d'azote =N–
$R^1$ représente l'atome d'hydrogène ou un radical alkyle,
$R^2$ représente un atome d'hydrogène ou un radical hydrocarboné éventuellement substitué.

EP 0 151 084 A2

La présente invention concerne de nouveaux produits, à usage phytosanitaire, à groupements triazole ou imidazole et oligoéther. L'invention concerne également des procédés de préparation desdits produits ainsi que leur application pour la protection des végétaux, spécialement dans le cadre de la lutte contre les champignons parasites mais aussi de la régulation de croissance des végétaux.

De nombreux produits à groupe triazole, notamment des fongicides, sont déjà connus. Un but de l'invention est de fournir de nouveaux produits offrant de nouvelles voies de traitement des végétaux. Un autre but de l'invention est de fournir des produits ayant une bonne activité notamment contre la rouille, l'oïdium, et plus spécialement contre l'oïdium des céréales. Un autre but de l'invention est de fournir des produits à activité polyvalente, cette activité s'étendant notamment à la pourriture grise (Botrytis) et la cercosporiose, au piétin-verse et aux maladies des semences. D'autres buts et avantages de l'invention apparaitront au cours de la description qui va suivre.

Il a maintenant été trouvé que ces buts pouvaient être atteints grâce aux produits de l'invention. Ceux-ci sont caractérisés en ce qu'ils ont pour formule :

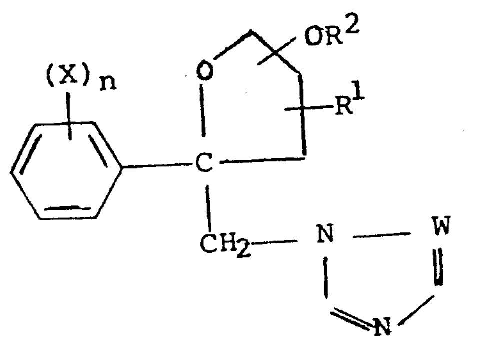

(I)

dans laquelle :

X est un atome d'halogène, de préférence le fluor, le brome ou le chlore, ou un groupe cyano ou nitro ou alkyle ou alkoxy ayant de 1 à 12 atomes de carbone, de préférence de 1 à 4 atomes de carbone, et étant éventuellement mono ou polyhalogéné (groupement $CF_3$ notamment),

n est un nombre entier, positif ou nul, inférieur à 6, de préférence égal à 2, étant entendu que lorsque n est supérieur à 1, les substituants X peuvent être soit identiques, soit différents,

W représente un groupe trivalent constitué soit d'un groupe =CH- soit d'un atome d'azote =N-

$R^1$ représente l'atome d'hydrogène ou un radical alkyle, ayant de préférence de 1 à 4 atomes de carbone,

$R^2$ représente un atome d'hydrogène ou un radical hydrocarboné éventuellement substitué ; comme radicaux plus particulièrement utilisables, on peut citer les radicaux alkyle, cycloalkyle, aryle et aralkyle ; comme substituants, on peut citer en particulier les atomes d'halogène, de préférence le chlore et le fluor, et les groupes alkoxy et aryloxy. Le groupe $OR^2$ est de préférence rattaché à l'atome de carbone vicinal de l'atome d'oxygène de l'hétérocycle de la formule (I), en sorte que le composé de formule (I) est alors pourvu d'une fonction acétal.

Parmi les composés répondant à la formule (I), les composés pour lesquels W représente un atome d'azote sont préférés pour les applications fongicides

Une sous-classe préférée de produits selon l'invention est donc constituée par les produits de formule

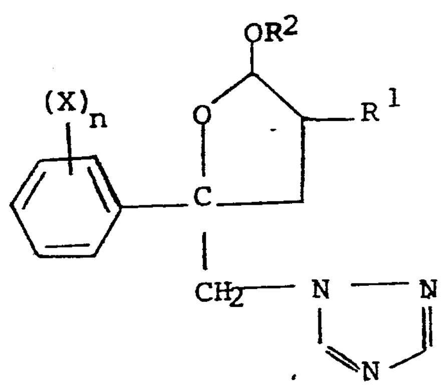

(Ia)

dans laquelle :

X est un atome d'halogène,

n est égal à 1, 2 ou 3. En vue de l'utilisation contre le piétin-verse, on préfère les composés dans lesquels n = 2.

Les composés selon l'invention peuvent exister sous une ou plusieurs formes d'isomères optiques selon le nombre de centres asymétriques de la molécule. L'invention concerne donc aussi bien ces isomères optiques que leurs mélanges racémiques et les diastéréoisomères correspondants. La séparation des diastéréoisomères et/ou des isomères optiques peut s'effectuer selon les méthodes connues en soi.

L'invention concerne également les formes salifiées des composés selon l'invention et plus spécialement les chlorhydrates, sulfates, oxalates, nitrates.

La présente invention concerne également des procédés de préparation des composés selon l'invention.

Selon un premier procédé, on fait réagir un composé de formule

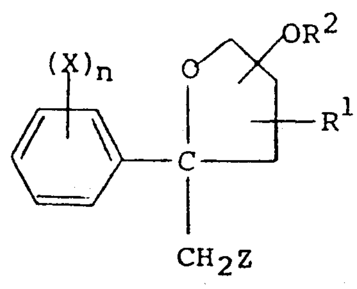

(II)

dans laquelle Z est un atome de chlore ou de brome, et X, n, $R^1$ et $R^2$ ont même signification que dans la formule (I), avec un dérivé alcalin (par exemple un sel de sodium ou de potassium), ou un dérivé d'ammonium ou phosphonium quaternaires) d'un imidazole ou triazole.

La réaction s'effectue habituellement en milieu solvant polaire aprotique et peut aussi être catalysée, par exemple par addition d'iodure alcalin ; la température est généralement comprise entre 50 et 250°C, de préférence entre 70 et 230°C. Pour des raisons économiques, les concentrations globales en réactifs comprises entre 1 et 50% sont le plus souvent utilisées.

Les composés de formule

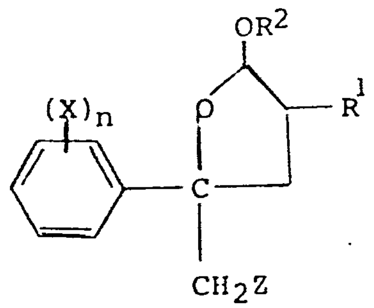

(IIa)

peuvent se préparer par réaction d'un alcool $R^2OH$ avec un composé de formule

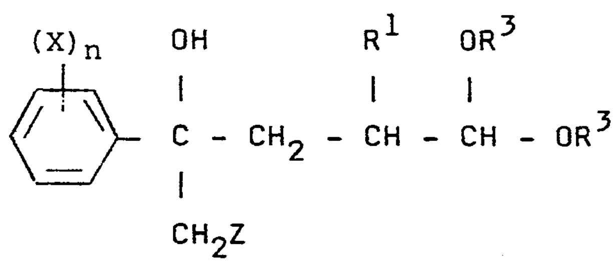

(III)

en présence d'un catalyseur acide, $R^1$, $R^2$, X, Z et n ayant les mêmes significations que précédemment et $R^3$ étant un radical organique, de préférence alkyle inférieur ($C_{1-4}$), deux radicaux $R^3$ pouvant constituer ensemble un radical organique divalent, de préférence alkylène inférieur.

L'acide catalyseur mis en oeuvre dans cette réaction peut être un acide protique ou aprotique. Comme acides protiques, on peut citer les acides chlorhydrique, sulfurique, trifluoroacétique, perchlorique, benzène-sulfonique, toluène-sulfonique, méthane-sulfonique. Comme acides aprotiques on peut citer les acides de Lewis tels que $BF_3$, $AlCl_3$ et $SnCl_4$. Lorsqu'on utilise, comme catalyseur, l'acide chlorhydrique, ce dernier peut être généré in situ, par exemple à l'aide de chlorure d'acyle, et notamment de chlorure d'acétyle, lequel réagit sur l'alcool présent pour donner naissance à HCl.

La réaction s'effectue ordinairement par simple chauffage des réactifs indiqués. La température est

généralement comprise dans la zone de température allant de 50°C à la température d'ébullition du milieu réactionnel. L'alcool $R^2OH$ joue habituellement le rôle de solvant dans le milieu réactionnel. Un cosolvant inerte peut être également ajouté, notamment un hydrocarbure aliphatique, alicyclique ou aromatique, halogéné ou non, ou un éther.

Les composés de formule (III) se préparent habituellement par réaction d'un composé carbonylé de formule

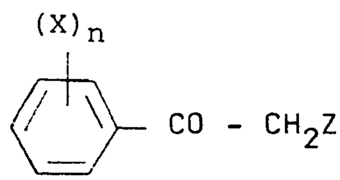

(IIIa)

avec le dérivé organomagnésien préparé à partir d'un composé de formule

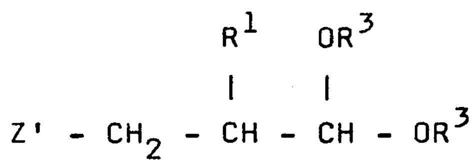

(IIIb)

dans lesquelles Z' est un atome d'halogène, de préférence le brome, et X, Z, $R^1$, $R^3$ et n ont les significations précédemment indiquées. Le dérivé organomagnésien peut être préparé, de manière connue en soi, par action d'un cétal de ß-halogénoaldéhyde sur le magnésium en milieu solvant. Ce cétal de ß-halogénoaldéhyde peut lui-même être préparé selon des méthodes connues, par exemple selon G. Büchi et H. Wüest, J. Org. Chem. 34 1122 (1969) et H. Meerwein, Houben-Weyl, Methoden der Org. Chem. vol VI/3 p 204, 4° Ed (1965).

La réaction du composé de formule (IIIa) avec le dérivé organomagnésien du composé de formule (IIIb) s'effectue le plus souvent à une température comprise entre

-70°C et +100°C, de préférence entre -50°C et +50°C. Comme solvant on peut citer les éthers, notamment l'éther diéthylique et le tétrahydrofuranne, ou les hydrocarbures aliphatiques, alicycliques ou aromatiques, ou leurs mélanges.

Selon un autre procédé de préparation de composés selon l'invention, on fait réagir un alcool $R^2OH$ avec un composé de formule

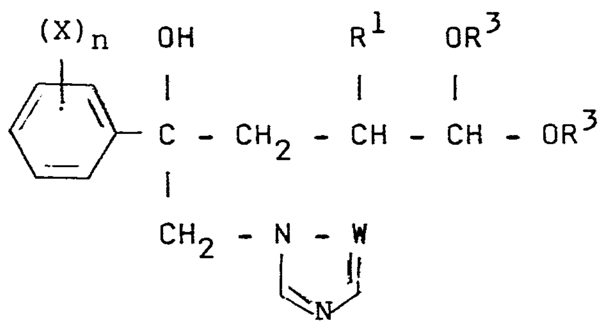

(IV)

en présence d'un catalyseur acide. Les divers symboles indiqués pour les formules de ces deux réactifs ont les significations données plus haut. Comme catalyseur acide on peut également utiliser ceux définis plus haut à propos de la préparation des composés de formule (IIa). Les autres conditions réactionnelles sont aussi semblables à celles définies pour la préparation de ces composés de formule (IIa).

Les composés de formule (IV) peuvent s'obtenir par réaction d'imidazole ou triazole avec un composé de formule

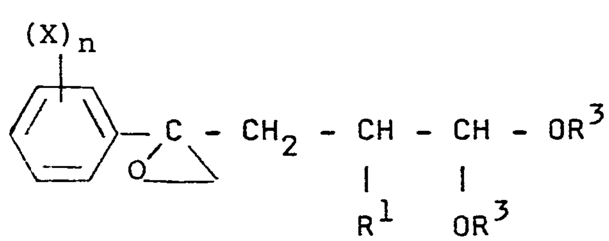

(V)

dans laquelle les divers symboles ont les significations

déjà indiquées.

L'imidazole ou triazole est éventuellement, totalement ou de préférence partiellement, sous forme de sel alcalin.

La réaction s'effectue habituellement à température comprise entre la température ambiante (20°C) et la température d'ébullition à reflux, de préférence entre +50 et +130°C. Comme solvants on peut utiliser notamment des alcools, des éthers, et des solvants polaires aprotiques tels que le diméthylformamide et le diméthylsulfoxyde.

Dans des conditions semblables à celles décrites par la préparation de composés de formule (IV) à partir de composés de formule (V), on peut aussi préparer ces composés de formule (IV) à partir de composés de formule (III) et à l'aide de dérivé alcalin d'imidazole ou triazole.

Les composés de formule (V) peuvent s'obtenir par déshydrohalogénation de composés de formule (III). Cette réaction s'effectue habituellement par action d'une base organique ou, de préférence, minérale, à température comprise entre 0 et 100°C. Le milieu peut être aqueux et/ou contenir un solvant tel qu'un hydrocarbure ou un éther, un alcool, un solvant polaire.

Les exemples suivants donnés à titre non limitatif illustrent l'invention et montrent comment elle peut être mise en oeuvre.

Les exemples 1 à 3 et 9 et le tableau (I) illustrent des modes particuliers de préparation de composés selon l'invention, ainsi que ces composés eux-mêmes. Parmi les propriétés physiques indiquées pour ces composés, on a indiqué les valeurs des déplacements (delta) en RMN du proton du groupe -O-CH-O- (acétal). Ces déplacements sont mesurés en ppm et ils sont repérés par rapport à un produit de référence qui est le tétraméthyl silane. La RMN est effectuée à 100 MHz dans le chloroforme deutéré.

Les exemples 4 à 7 illustrent les propriétés fongicides des composés selon l'invention ainsi que leurs applications.

Dans ces exemples, les pulvérisations de solutions ou suspensions de matières actives sont effectuées dans des conditions telles que la pulvérisation d'une solution ou suspension de concentration égale à 1 g/l correspond en moyenne à l'application d'environ 2 microgrammes de matière active par $cm^2$ de feuille de la plante.

Dans les conditions des exemples 4 à 7, les composés illustrés n'ont pas présenté de phytotoxicité.

Dans ces exemples on considère qu'un produit exerce une protection totale vis-à-vis d'une maladie fongique lorsque la protection est d'au moins 95 % ; la protection est considérée comme bonne lorsqu'elle est d'au moins 80 % (mais inférieure à 95 %), comme assez bonne lorsqu'elle est d'au moins 70 % (mais inférieure à 80 %), comme moyenne lorsqu'elle est d'au moins 50 % (mais inférieure à 70 %).

Dans le présent exposé les pourcentages sont, sauf indication contraire et sauf ceux concernant les rendements, des pourcentages pondéraux. Dans le cas où les pourcentages sont exprimés par rapport à la stoechiométrie, il s'agit de pourcentages molaires. En ce qui concerne les concentrations, certaines d'entre elles sont exprimées en ppm (partie par millions) ce qui correspond à des mg/l.

<u>Exemple 1</u> :

On prépare un dérivé organomagnésien par activation de 9,7 g de magnésium (0,4 mole) par 0,5 ml de dibromoéthane dans 10 ml de tétrahydrofurane anhydre (THF en abrégé). En maintenant la température inférieure à 15°C, on ajoute goutte à goutte une solution de 47 ml (0,4 mole) de β-bromoéthyl-2 dioxolanne-1,3 dans 200 ml de THF. Un quart d'heure après la fin de l'addition on refroidit à -45°C et ajoute une solution de 56,7 g (0,3 mole) de chlorométhyl-(parachlorophényl)cétone dans 150 ml de THF, la température étant maintenue à -45°C. Après une

demi-heure le milieu est neutralisé à l'aide de 120 ml d'acide acétique pur, puis versé dans un litre d'eau. On opère une extraction à l'aide d'acétate d'éthyle. La solution dans l'acétate d'éthyle est séchée, le solvant évaporé. On obtient 100 g de chlorhydrine fondant à 99°C (après recristallisation dans le cyclohexane) et de formule

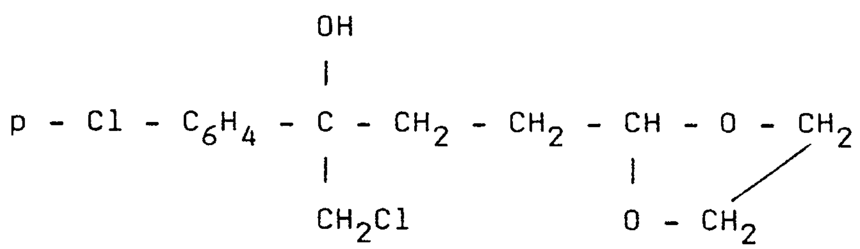
(VI)

On chauffe 2 h à l'ébullition à reflux un mélange de 0,1 ml de chlorure d'acétyle et d'une solution de 6 g (0,02 mole) du produit de formule (VI) dans 30 ml de méthanol. Le mélange réactionnel est alors versé dans une solution aqueuse à 5 % en poids de carbonate de sodium dans l'eau. On extrait à l'aide d'éther éthylique, sèche la solution éthérée et évapore l'éther. On obtient une huile qu'on distille à 140°C sous une pression absolue de 0,04 mmHg et on obtient 4,7 g de produit huileux de formule :

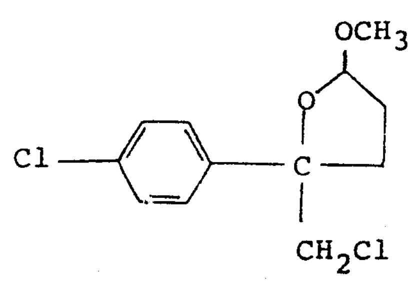
(VII)

On chauffe 3 h à 170°C sous atmosphère inerte un mélange obtenu par addition de 4,2 g (0,016 mole) de produit de formule (VII) à 40 ml de diméthylsulfoxyde contenant du sel de sodium de triazole préparé à partir de 1,6 g (0,024 mole) de triazole et 0,7 g (0,024 mole) d'hydrure de sodium en suspension huileuse à 80 %.

La solution est versée dans 200 ml d'eau. On

extrait à l'aide d'éther éthylique, sèche la solution éthérée, évapore. Le résidu est purifié par chromatographie sur colonne de silice. On obtient ainsi 3,1 g d'une huile constituée d'un mélange en proportions sensiblement égales de deux diastéréoisomères de formule développée :

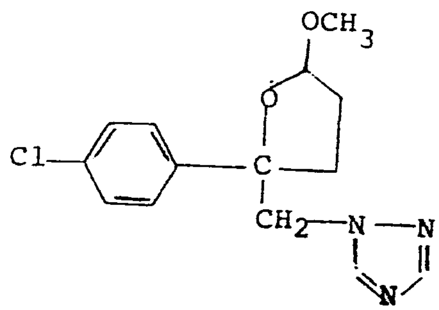

(VIII)

Exemple 2 :

On prépare le produit de formule :

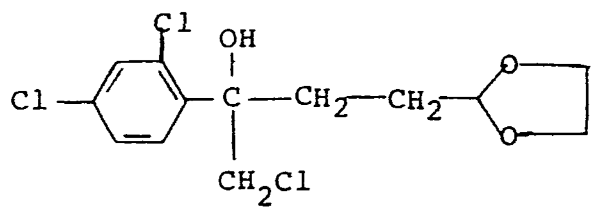

(IX)

par un procédé semblable à celui permettant la préparation du produit de formule (VI) à l'exemple 1, mais en utilisant comme réactif la chlorométhyl-(orthoparadichlorophényl)cétone au lieu de la chlorométhyl-(parachlorophényl)cétone.

On agite 12 h à température ambiante un mélange de 0,3 mole de produit de formule (IX) avec 400 ml de solution aqueuse d'hydroxyde de sodium de concentration pondérale égale à 15 %. La phase organique est diluée à l'éther éthylique, séparée par décantation, lavée à l'eau, séchée et évaporée.

On obtient par distillation 64 g d'une huile visqueuse incolore de point d'ébullition 147 à 150°C sous une pression absolue réduite à 0,02 mmHg et ayant pour

formule :

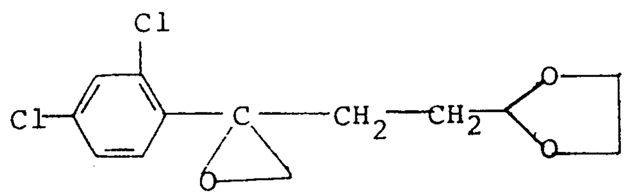

(X)

On chauffe 6 h à 110°C un mélange de :

- une solution de 5,8 g (0,02 mole) de produit de formule (X) dans 20 ml de n-butanol,

- 1,4 g (0,02 mole) de triazole,

- 0,9 g (0,01 mole) de sel de sodium de triazole.

On refroidit à température ambiante, on dilue à l'eau, extrait à l'éther puis concentre la solution éthérée. Le résidu est purifié par chromatographie sur colonne de silice en utilisant comme éluant un mélange méthanol/acétate d'éthyle/hexane en proportions volumiques respectives 5 / 47,5 / 47,5.

On obtient 5,1 g de produit fondant à 131°C et ayant pour formule :

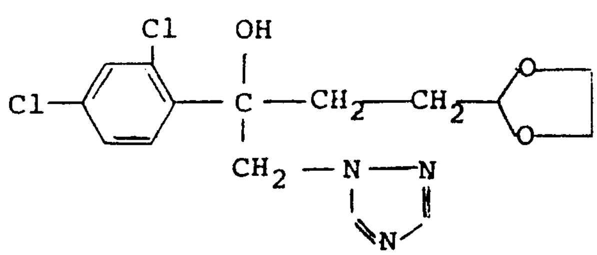

(XI)

On chauffe 4 h à l'ébullition à reflux un mélange de 0,10 ml de chlorure d'acétyle avec une solution de 3,1 g de produit de formule (XI) dans 30 ml d'éthanol absolu. On laisse refroidir. Les cristaux formés sont filtrés, lavés avec de l'éthanol froid. On obtient ainsi 1,2 g de cristaux blancs, fondant à 162°C et correspondant au diastéréoisomère le plus polaire (observé en chromatographie en couche mince) du produit de formule

développée :

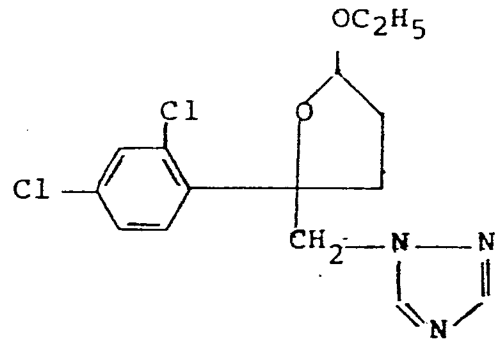

(XII)

Les eaux mères issues de cette filtration sont concentrées, le résidu est dilué avec 1 ml d'éthanol puis 2 ml d'oxyde d'isopropyle et on sépare les cristaux formés. On obtient encore 0,2 g du même diastéréoisomère fondant à 164°C.

Par chromatographie sur silice de l'huile résiduelle de la cristallisation, on obtient un second diastéréoisomère, le moins polaire, qui fond à 61°C.

Exemple 3 :

En utilisant des procédés similaires aussi bien à celui de l'exemple 1 qu'à ceux des exemples 2 et 9 on a préparé divers autres composés ayant pour formule :

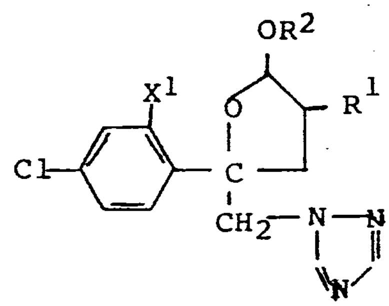

(XIII)

La nature des substituants dans cette formule (XIII) et certaines caractéristiques physiques des composés 1 à 50 sont indiquées dans le tableau (I), qui inclut aussi les produits préparés (n° 1, 10, 11 et 29) aux exemples 1, 2 et 9.

Toutefois, en ce qui concerne les composés n°49 et 50, au lieu de comporter un groupe parachlorophényle substitué en ortho par $X^1$, ils comportent un groupe parafluorophényle substitué en ortho par $X^1$. De plus le

composé n°50 a un groupe imidazole au lieu de triazole, le procédé de préparation restant le même, mais les réactifs étant convenablement choisis.

Dans le cas où $R^1$ est l'atome d'hydrogène, les composés comportent 2 atomes de carbone asymétriques et les deux diastéréoisomères peuvent être distingués par chromatographie sur couche mince de silice en utilisant comme éluant un mélange méthanol/acétate d'éthyle/hexane en proportions volumiques respectives 5/20/75. Le moins polaire migre le plus haut en chromatographie et est appelé A. Le plus polaire migre le plus bas en chromatographie et est appelé B.

Dans le cas où $R^1$ est différent de l'atome d'hydrogène, les composés comportent 3 atomes de carbone asymétriques et il y a 4 diastéréoisomères. Ces diastéréoisomères sont dénommés arbitrairement A, B, C et D. Ils sont quelquefois difficiles à séparer par chromatographie mais lorsqu'ils peuvent l'être, A est le moins polaire, D le plus polaire, B et C ont des polarités intermédiaires.

Exemple 4 :

Test in vivo sur Erysiphe graminis sur orge (oïdium de l'orge)

On prépare par broyage fin une émulsion aqueuse de la matière active à tester ayant la composition suivante :

- matière active à tester 40 mg
- Tween 80 (agent tensioactif constitué d'un oléate de dérivé polyoxyéthylèné du sorbitan) dilué à 10 % dans l'eau 0,4 ml
- eau 40 ml.

Cette émulsion aqueuse est ensuite diluée par de l'eau pour obtenir la concentration désirée.

De l'orge, en godets, semée dans de la terre franche, est traitée au stade 10 cm de hauteur par pulvérisation d'une émulsion aqueuse (appelée bouillie) de concentration indiquée ci-après. L'essai est répété deux

fois. Au bout de 48 heures, on saupoudre les plants d'orge avec des spores d'*Erysiphe graminis*, le saupoudrage étant effectué à l'aide de plants malades.

La lecture se fait 8 à 12 jours après la contamination.

Dans ces conditions, on observe les résultats suivants :

A la dose de 1 g/l, protection bonne ou totale avec les composés

1, 2, 4, 5, 6, 7, 8, 17, 18, 19, 20 .

A la dose de 0,33 g/l, protection totale avec les composés

8, 14, 16, 27.

A la dose de 0,11 g/l, protection totale avec les composés

11, 12, 13, 15, 28.

A la dose de 0,033 g/l, protection totale avec les composés

9 et 10.

Exemple 5 :

Test in vivo sur "Puccinia recondita" responsable de la rouille du blé

Du blé, en godets, semé dans de la terre franche, est traité au stade 10 cm de hauteur par pulvérisation avec des émulsions aqueuses (appelées bouillies) de même composition que celle décrite à l'exemple 4 et à diverses concentrations du composé à tester. L'essai est répété deux fois avec chaque concentration.

Au bout de 48 heures, une suspension aqueuse de spores (50000 sp/$cm^3$) est pulvérisée sur le blé ; cette suspension a été obtenue à partir de plants contaminés. On place ensuite le blé pendant 48 heures en cellule d'incubation à environ 18°C et à 100 % d'humidité relative.

Au bout de ces 2 jours, l'humidité relative est ramenée à 60 %. Le contrôle de l'état des plants se fait entre le 11ème et le 15ème jour après la contamination par comparaison avec le témoin non traité.

Dans ces conditions, on observe les résultats suivants :

A la dose de 1 g/l, bonne protection avec les composés 4, 5, 6, 7, 9, 13, 14, 15, 16, 17, 18, et 21.

A la dose de 0,33 g/l, protection totale avec les composés 10 et 11.

Exemple 6 :

Test sur Botrytis cinerea sur tomate :

Des tomates cultivées en serre (variété Marmande) agées de 30 à 40 jours sont traitées par pulvérisation avec des émulsions aqueuses (appelées bouillies) de même composition que celle décrite à l'exemple 4 et à diverses concentrations du composé à tester. L'essai est répété deux fois avec chaque concentration.

Après 24 ou 48 heures, les feuilles sont coupées et mises dans 2 boîtes de Pétri (diamètre 11cm) dont le fond a été préalablement garni d'un disque de papier filtre humide (5 folioles par boîte).

L'inoculum est ensuite apporté à l'aide d'une seringue par dépôt de gouttes (3 gouttes par foliole) d'une suspension de spores. Cette suspension de spores de Botrytis cinerea a été obtenue à partir d'une culture de 15 jours, mise ensuite en suspension dans une solution nutritive (80.000 unités/$cm^3$).

Le contrôle est fait 3 jours après la contamination par comparaison avec un témoin non traité.

Dans ces conditions on observe, à la dose de 1 g/l, une protection bonne ou totale avec les composés n° 8, 10 et 16.

Exemple 7 :

Tests in vitro sur champignons des semences et champignons du sol

On étudie l'action des composés selon l'invention sur les champignons suivants responsables des maladies secondaires des céréales :

Cercosporella herpotrichoïdes (CERC)

Helminthosporium gramineum (HELM G)

| | |
|---|---|
| Pyrenophorae avenae | (PYRE) |
| Septoria nodorum | (SEPT N) |
| Helminthosporium teres | (HELM T) |
| Fusarium roseum | (FUS ROS) |
| Fusarium nivale | (FUS NIV) |
| Fusarium culmorum | (FUS CULM) |
| Rhizoctonia cerealis | (RHIZ C) |

Les indications figurant entre parenthèses seront utilisées pour représenter ces champignons dans le tableau (II).

Pour chaque essai, on opère de la manière suivante: un milieu nutritif constitué de pomme de terre, de glucose et de gelose (milieu PDA) est introduit en surfusion dans une série de boîtes de Petri (20 ml par boîte) après stérilisation à l'autoclave à 120°C.

Au cours du remplissage des boîtes, on injecte, dans le milieu en surfusion, une solution acétonique de la matière active, pour obtenir la concentration finale désirée.

On prend comme témoin des boîtes de Pétri analogues aux précédentes, dans lesquelles on coule des quantités similaires d'un milieu nutritif ne contenant pas de matière active.

Après 24 ou 48 h chaque boîte est ensemencée par dépôt d'un fragment de mycelium provenant d'une culture précédente du même champignon.

Les boîtes sont conservées pendant 2 à 10 jours (selon le champignon testé) à 22°C et on compare alors la croissance du champignon dans les boîtes contenant la matière active à tester, à celle du même champignon dans la boîte utilisée comme témoin.

On détermine ainsi pour chaque composé testé la plus faible dose permettant d'inhiber à 80-100 % le développement du champignon considéré. Cette dose est appelée "Dose minimale inhibitrice".

Ces doses minimales inhibitrices, exprimées en ppm,

sont rapportées dans le tableau (II), dans lequel les abréviations ont la signification indiquée plus haut.

Les composés selon l'invention peuvent donc être utilisés pour la lutte tant préventive que curative contre les champignons, notamment de type basidiomycètes, ascomycètes, adelomycètes ou fungi-imperfecti, en particulier les rouilles, oïdium, piétin-verse, fusarioses, helminthosporioses, septorioses, rhizoctones des végétaux et des plantes en général et en particulier des céréales telles que le blé, l'orge, le seigle, l'avoine et leurs hybrides et aussi le riz et le maïs.

Les produits de l'invention sont spécialement intéressants par leur spectre large au niveau des maladies des céréales (oïdium, rouille, piétin-verse, helminthosporioses, septorioses et en particulier les fusarioses difficiles à combattre). Ils présentent également un grand intérêt en raison de leur activité sur la pourriture grise (Botrytis) et les cercosporioses, et, de ce fait, ils peuvent être appliqués sur des cultures aussi variées que la vigne, les cultures maraîchères et l'arboriculture

Ils présentent enfin une excellente sélectivité vis-à-vis des cultures.

Ils s'appliquent avantageusement à des doses de 0,02 à 5kg/ha, de préférence de 0,05, plus spécifiquement de 0,1, à 2kg/ha.

Pour leur emploi pratique, les composés selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un composé selon l'invention tel que décrit précédemment en association avec les supports solides ou liquides, acceptables en agriculture et les agents tensio-actifs également acceptables en agriculture. En

particulier sont utilisables les supports inertes et usuels et les agents tensio-actifs usuels.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Ces doses d'emploi dans le cas d'une utilisation comme fongicides des composés selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5\times10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

Selon ce qui a déjà été dit les composés selon l'invention sont généralement associés à des supports et éventuellement des agents tensioactifs.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par

granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,01 à 20 % de matière active. En plus du solvant, les concentrés émulsionnables peuvent contenir, quand c'est nécessaire, 2 à 20 % d'additifs appropriés, comme des stabilisants, des agents tensioactifs, des agents de pénétration, des inhibiteurs de corrosion, des colorants, des adhésifs. A titre d'exemple, voici la composition de quelques concentrés :

Exemple F (formulation) 1

| | | |
|---|---|---|
| - matière active | | 400 g/l |
| - dodécylbenzène sulfonate alcalin | | 24 g/l |
| - nonylphénol oxyéthylé à 10 molécules d'oxyde d'éthylène | | 16 g/l |
| - cyclohexanone | | 200 g/l |
| - solvant aromatique | q.s.p | 1 litre |

Selon une autre formule de concentré émulsionnable, on utilise :

Exemple F 2 :

| | |
|---|---|
| - matière active | 250 g |
| - huile végétale époxydée | 25 g |
| - mélange de sulfonate d'alcoylaryle et d'éther de polyglycol et d'alcools gras | 100 g |
| - diméthylformamide | 50 g |
| - xylène | 575 g |

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

Exemple F 3 :

| | |
|---|---|
| - matière active | 50 % |
| - lignosulfonate de calcium (défloculant) | 5 % |
| - isopropylnaphtalène sulfonate (agent mouillant anionique) | 1 % |
| - silice antimottante | 5 % |
| - kaolin (charge) | 39 % |

Une autre composition de poudre à pulvériser à 70 % utilise les constituants suivants :

Exemple F 4 :

| | |
|---|---|
| - matière active | 700 g |
| - dibutylnaphtylsulfonate de sodium | 50 g |
| - produit de condensation en proportions 3/2/1 d'acide naphtalène sulfonique, d'acide phénolsulfonique et de formaldéhyde | 30 g |
| - kaolin | 100 g |
| - craie de champagne | 120 g |

Une autre composition de poudre à pulvériser à 40 % utilise les constituants suivants :

Exemple F 5 :

| | |
|---|---|
| - matière active | 400 g |
| - lignosulfonate de sodium | 50 g |
| - dibutylnaphtalène sulfonate de sodium | 10 g |
| - silice | 540 g |

Une autre composition de poudre à pulvériser à 25 % utilise les constituants suivants :

Exemple F 6 :

| | |
|---|---|
| - matière active | 250 g |
| - lignosulfonate de calcium | 45 g |
| - mélange équipondéral de craie de Champagne et d'hydroxyéthylcellulose | 19 g |
| - dibutylnaphtalène sulfonate de sodium | 15 g |
| - silice | 195 g |
| - craie de Champagne | 195 g |
| - kaolin | 281 g |

Une autre composition de poudre à pulvériser à 25 % utilise les constituants suivants :

Exemple F 7 :

| | |
|---|---|
| - matière active | 250 g |
| - isooctylphénoxy-polyoxyéthylène-éthanol | 25 g |
| - mélange équipondéral de craie de Champagne et d'hydroxyéthylcellulose | 17 g |

- aluminosilicate de sodium 543 g
- kieselguhr 165 g

Une autre composition de poudre à pulvériser à 10 % utilise les constituants suivants :

Exemple F 8 :

- matière active 100 g
- mélange de sels de sodium de sulfates d'acides gras saturés 30 g
- produit de condensation d'acide naphtalène sulfonique et de formaldéhyde 50 g
- kaolin 820 g

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être

fabriqués par agglomération ou imprégnation. En général, les granulés contiennent 0,5 à 25 % de matière active et 0 à 10 % d'additifs comme des stabilisants, des agents de modification à libération lente, des liants et des solvants.

Selon un exemple de composition de granulé, on utilise les constituants suivants :

Exemple F 9 :

| | |
|---|---|
| - matière active | 50 g |
| - épichlorhydrine | 2,5 g |
| - éther de cétyle et de polyglycol | 2,5 g |
| - polyéthylène glycol | 35 g |
| - kaolin (granulométrie : 0,3 à 0,8 mm) | 910 g. |

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) peuvent encore être utilisés sous forme de poudres pour poudrage ; on peut aussi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

D'autres exemples ont encore été réalisés. Dans ces exemples on a utilisé les abréviations suivantes :

| | |
|---|---|
| Botrytis cinerea | BOT |
| Erysiphe graminis | ERYG |
| Puccinia recondita | PUCR |
| Plasmopara viticola | PLA |
| Piricularia oryzae | PIR |
| Cercospora beticola | CBET |
| Peronospora tabacina | PERO |

| | |
|---|---|
| Puccinia striiformis | PUCS |
| Erysiphe cichoracearum | ERYC |
| Fusarium oxysporum (meloni) | FUS OX |
| Pythium spp | PYT |
| Pyrenophora avenae | PYR |
| Septoria tritici | SEPT T |
| Venturia inequalis | VENT |
| Whetzelinia sclerotiorum | WHE |
| Monilia laxa | MON |
| Mycosphaerella fijiensis | MSPH |
| Marssonina panattoniana | MARS |
| Alternaria solani | ALT |
| Aspergillus niger | ASP |
| Cercospora arachidicola | CARA |
| Cladosporium herbarum | CLAD |
| Helminthosporium oryzae | HELM OR |
| Penicillium expansum | PEN |
| Pestalozzia sp | PES |
| Phialophora cinerescens | PHI |
| Phoma betae | PHB |
| Phoma foveata | PHF |
| Phoma lingam | PHL |
| Ustilago maydis | UST |
| Verticillium dahliae | VERT |
| Ascochyta pisi | ASCO |
| Guignardia bidwellii | GUIG |
| Corticium rolfsii | CRO |
| Phomopsis viticola | PHV |
| Sclerotinia sclerotiorum | SCL S |
| Sclerotinia minor | SCL M |
| Phytophthora cinnamomi | PHY CI |
| Phytophthora cactorum | PHY CC |
| Phytophthora capsici | PHY CP |
| Phytophthora infestans | PHY IN |
| Phytophthora parasitica | PHY PA |
| Phytophthora megasperma | PHY ME |

| | |
|---|---|
| Phytophthora syringae | PHY SY |
| Coryneum cardinale | CORY |
| Rhizoctonia solani | RHIZ S |

Le signe > signifie "plus grand que"
Le signe ≤ signifie "inférieur ou egal à"

Le piétin-verse est désigné plus haut par Cercosporella herpotrichoides, mais il est normalement appelé Pseudocercosporella herpotrichoides.

Les tableaux (III), (IV) et (VI) présentent la dose minimale inhibitrice in vitro de divers composés à l'égard de divers champignons selon la méthode d'application utilisée à l'exemple 7.

Le tableau (V) présente la dose minimale inhibitrice en serre (in vivo) de divers composés à l'égard de divers champignons selon les méthodes d'applications utilisées dans les exemples 4 à 6.

Le tableau (VII) présente l'efficacité des produits en plein champs vis à vis de divers composés.

On donne ci-après la nature du test utilisé en serre (in vivo) vis à vis de Cercospora beticola (Exemple 8) et les conditions des tests en plein champ (Exemple 8 bis).

Exemple 8 :

Des plantules de betteraves à sucre de 7 à 10 cm de hauteur sont traitées préventivement par la bouillie à tester, cette bouillie étant semblable à celle décrite à l'exemple 4. L'essai est répété 2 fois à chaque concentration.

24 heures après traitement, les plantules de betteraves sont contaminées par pulvérisation d'une suspension aqueuse à 0,2 g/ml de mycelium de Cercospora Beticola.

On laisse incuber pendant 72 h à 25°C et 100 %

d'humidité relative puis au bout de 3 jours on place les plantules sous lumière (10000 lux ; 14 heures par jour). Le contrôle de l'état des plantes se fait 14 jours après contamination et s'exprime en pourcentage par rapport à un témoin non traité. Un effet identique au témoin est noté 0 %.

Une protection complète de la plante est notée 100 %.

Exemple 8 bis :

Les conditions particulières des différents tests sont indiquées dans le tableau (VII). Les conditions générales sont les suivantes : Les tests ont été répétés 4 fois chacun, sur des parcelles de terrain de 3 à 5 $m^2$. Les contaminations sont naturelles pour l'oïdium (ERYG), artificielles pour le piétin-verse (CERC) et la rouille jaune (PUCR). Les plantes ont été traitées par une bouillie répandue à raison de 500 à 1000 l/ha sous une pression de 3 $kg/cm^2$.

Le traitement était préventif pour l'oïdium et la rouille jaune, (traitement répété tous les 15 jours), curatif pour le piétin-verse. Les résultats ont été observés sur un prélèvement de 25 feuilles pour l'oïdium et la rouille jaune (détermination du pourcentage de surface foliaire atteinte par la maladie), ou sur un prélèvement de 25 tiges pour le piétin-verse (détermination du pourcentage de tiges atteintes).

Exemple 9 :

On refroidit à 0°C une solution de 296 g (4,23 moles) de méthacroléine dans 1 l de dichlorométhane. On fait barboter 343 g d'HBr gazeux en maintenant la température. Après 1/4 d'heure on ajoute 0,9 l de méthanol à 0°C, température à laquelle le mélange est maintenu pendant 3 h. On ajoute 2 l d'eau contenant 100 ml d'ammoniaque (aqueux) concentré. Le mélange est décanté, la phase organique est séparée et la phase aqueuse extraite avec 200 ml de dichlorométhane. Les phases organiques sont combinées, lavées avec une solution aqueuse de bisufite de sodium ; on concentre, puis distille en présence de

diéthylaniline. On obtient 452 g de bromoacétal de formule

$Br-CH_2-CH(CH_3)-CH(OCH_3)_2$ - Rendement : 54 % ; point d'ébullition 44 à 50°C sous pression réduite à 4 mmHg.

On prépare alors un dérivé magnésien par addition d'une solution de 19,7 g de bromoacétal dans 40 ml de tétrahydrofuranne, goutte à goutte sur du magnésium, entre 15 et 22 °C et en présence de quelques gouttes de dibromo-1,2 éthane.

On ajoute alors 40 ml de tétrahydrofuranne, refroidit à -20°C puis ajoute goutte à goutte une solution de 20,1 g de trichloroacétophénone dans 50 ml de THF. La température est maintenue encore à -20°C. On ajoute 6 ml d'acide acétique et verse le mélange dans 500 ml d'eau à température ambiante. On extrait à l'éther ; la solution organique est séchée et concentrée de manière à obtenir 30 g d'huile jaune constituée essentiellement de chlorhydrine de formule

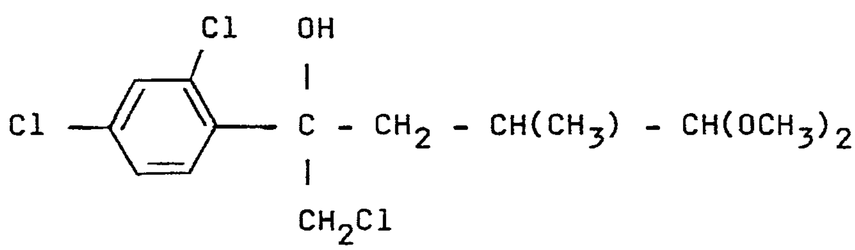

(Ce mode opératoire de préparation d'acétal non cyclique est celui qui permet l'accès aux composés de l'exemple 3 dans lesquels $R^1$ est autre que l'atome d'hydrogène).

Dans une solution de 442 g de chlorhydrine dans 1,5l d'éthanol, on verse une solution de 1 mole de KOH dans 200 ml de méthanol, jusqu'à ce que le milieu devienne basique. On concentre sous vide, dilue à l'éther, lave à l'eau, sèche sur sulfate de sodium, évapore, distille sous pression réduite (121 à 140°C sous 0,01 mm de mercure). On obtient 138 g de mélange de deux diastéréoisomères de

formule générale

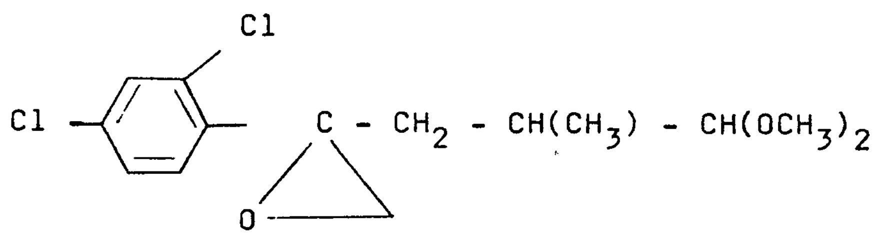

A une solution de 126,5 g de cet époxyde dans 500 ml de diméthylformamide on ajoute 57,2 g de triazole puis 172 g de $K_2CO_3$. On chauffe à 120°C pendant 4 h, filtre, lave avec du diméthylformamide et évapore. Le résidu est versé dans 2 l d'eau, extrait avec $CHCl_3$, lavé à l'eau, séché sur $Na_2SO_4$, évaporé sous vide. On obtient 148,2 g d'huile brun clair qui est triturée dans 100 ml d'heptane. On filtre et obtient 117,5 g de poudre beige fondant à 117°C (rendement : 78 %) et constitué du mélange des deux diastéréoisomères de formule

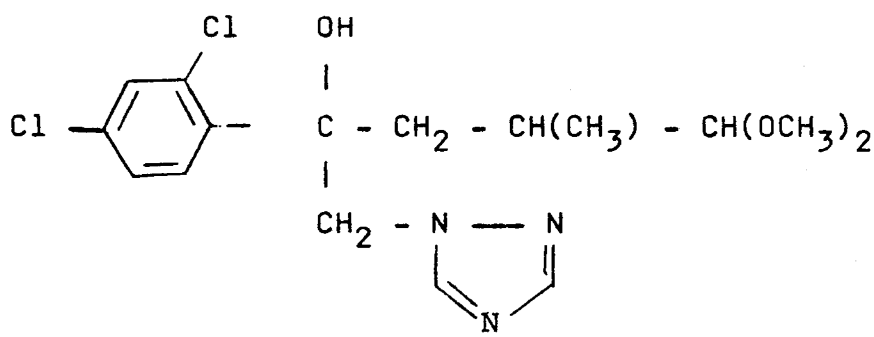

Les déplacements (delta) en RMN à 100 mégahertz dans le chloroforme deutéré sont repérés pour les protons du groupe méthoxy. On observe des déplacements à 3,23 et 3,19 ppm pour un diastéréoisomère et à 3,38 et 3,20 ppm pour l'autre diastéréoisomère.

A une solution de 75 g de cet hydroxytriazole dans 700 ml d'éthanol on ajoute 7,3 g d'HCl gazeux puis chauffe 15 heures à l'ébullition à reflux. L'éthanol est évaporé sous vide ; le résidu est repris dans l'acétate d'éthyle,

lavé à l'aide d'une solution aqueuse de bicarbonate, lavé à l'eau, séché sur $Na_2SO_4$. On évapore le solvant. On obtient 68,6 g d'huile brun clair. Rendement 96 % de produit de formule

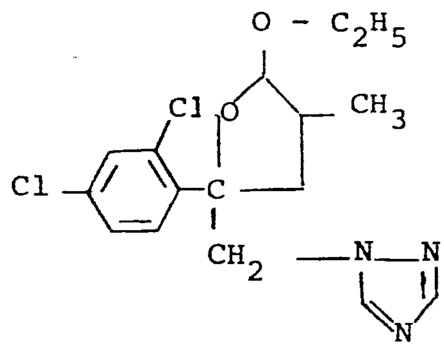

Parmi les produits de formules (I) et (Ia) selon l'invention on préfère les composés dans lesquels $R^1$ est $R^{10}$, le radical $R^{10}$ étant un radical alkyle. Ces composés constituent une classe ayant des propriétés particulièrement avantageuses et sont un objet particulier de la présente invention. De manière correspondante à ces composés, les produits intermédiaires de formule (II), (IIa), (III), (IIIb), (IIIc), (IV) et (V) dans lesquels $R^1$ est $R^{10}$, constituent aussi une classe de composés objet de la présente invention. De même les procédés permettant de préparer ces divers composés, selon les voies précédemment décrites mais en choisissant $R^{10}$ à la place de $R^1$ sont aussi un objet particulier de l'invention.

De manière encore plus préférentielle, dans ces divers composés et procédés, $R^{10}$ représente un radical alkyle inférieur ($C_1$ à $C_4$), et plus spécifiquement encore un radical méthyle.

Les divers procédés décrits plus haut s'appliquent aux divers composés de formule (I) à (V) aussi bien lorsque $R^1$ est l'atome d'hydrogène que lorsque $R^1$ est $R^{10}$. Toutefois la préparation du composé de formule (III) à partir de réactifs de formule (IIIa) et (IIIb) diffère selon que $R^1$ est H ou que $R^1$ est $R^{10}$ : lorsque $R^1$ est l'atome d'hydrogène, le réactif de formule (IIIb) est

un réactif de formule

$$Z' - CH_2 - CH_2 - CH(OR^3)_2 \qquad (IIIb1)$$

dans laquelle Z' à la signification déjà indiquée et les deux radicaux $R^3$ peuvent ou bien être distincts (identiques ou différents), ou bien (et cela est préféré) constituer un radical unique divalent ; lorsque $R^1$ est $R^{10}$, le réactif de formule (IIb) a pour formule

$$Z' - CH_2 - \underset{\displaystyle R^{10}}{\underset{|}{CH}} - \underset{\displaystyle OR^{30}}{\underset{|}{CH}} - OR^{30} \qquad (IIIb2)$$

dans laquelle Z' et $R^{10}$ ont les significations déjà indiquées et les deux radicaux $R^{30}$ ont les mêmes significations que celles données pour $R^3$ sauf que les deux radicaux R doivent être distincts (et non pas constituer un radical unique divalent). La référence H. Wüest, J. Org. Chem. 34 1122 (1969) concerne des produits de formule (IIIb) dans laquelle les deux radicaux $R^3$ constituent un radical unique divalent. La référence Houben-Weyl vol. VI/3 P. 204, 4e Ed, 1965 correspond à des produits de formule (IIIb2) dans laquelle les deux radicaux $R^{30}$ sont distincts.

Les produits de formules (I) et (Ia) dans lesquels $R^2$ est un radical alkyle halogéné, notamment chloré ou fluoré constituent aussi un objet de l'invention.

L'application des divers composés de formules (I), (Ia), dans lesquelles $R^1$ et $R^2$ et les autres substituants ont les diverses significations indiquées plus haut, à la lutte contre les atteintes fongiques des céréales, constitue encore un autre objet de l'invention.

Outre les applications déjà décrites plus haut, les produits selon l'invention présentent en outre une excellente activité biocide à l'égard de nombreuses autres

variétés de microorganismes parmi lesquelles on peut citer à titre non limitatif, des champignons comme ceux des genres :

- Pullularia comme l'espèce P. Pullulans,
- Chaetomium comme l'espèce C. Globosum,
- Aspergillus comme l'espèce Aspergillus niger,
- Coniophora comme l'espèce C. Puteana,

En raison de leur activité biocide, les produits de l'invention permettent de combattre efficacement les microorganismes dont la prolifération crée de nombreux problèmes dans les domaines agricole et industriel. A cet effet, ils conviennent tout spécialement bien à la protection des végétaux ou de produits industriels tels que le bois, le cuir, les peintures, le papier, les cordages, les plastiques, les circuits d'eau industriels;

Ils sont tout particulièrement bien adaptés à la protection des produits lignocellulosiques et notamment du bois, qu'il s'agisse de bois d'ameublement, de charpente ou de bois exposé aux intempéries tels que les bois de clôture, les piquets de vignes, les traverses de chemin de fer.

Dans les compositions de l'invention, les produits selon l'invention utilisés dans les traitements du bois peuvent être éventuellement associés à un ou plusieurs produits biocides connus tels que le pentachlorophénol, les sels métalliques, notamment de cuivre, de manganèse, de cobalt, de chrome, de zinc dérivés d'acides minéraux ou carboxyliques (acides heptanoïque, octanoïque, naphténique); les complexes organiques de l'étain, le mercaptobenzothiazole.

L'exemple n°10 suivant illustre l'activité biocide de composés selon l'invention.

<u>Exemple 10</u> :

On prépare une microsuspension du produit pour

déterminer son seuil fongistatique. Cette microsuspension est préparée de la manière suivante :

Dans un appareil de broyage à billes, on charge par tube :

- 6 ml d'une solution aqueuse contenant 2 agents mouillants (4 % de polyglycol 400 et 0,4 % de Tween 80)
- 5 g de billes en verre de diamètre 3 mm
- 3 g de billes en verre de diamètre 5 mm.

On soumet cette charge à une agitation jusqu'à obtention d'une microsuspension homogène.

Des quantités déterminées de microsuspension ainsi obtenue sont introduites dans un milieu de culture gélosé contenu dans des tubes à hémolyse. Chaque milieu est ensuite ensemencé par une souche déterminée d'un champignon. Pour chaque culture, on fait varier la quantité de produit biocide. Le seuil fongistatique est exprimé par la quantité de produit biocide pour 100 ml de milieu de culture à partir de laquelle on note une influence du produit sur le développement de la souche.

Les mélanges ont été classés en cinq catégories d'efficacité fongistatique croissante :

<u>Classe</u>

0 totalement inefficace à $1.10^{-2}$
$0^+$ efficacité voisine de $1.10^{-2}$
1 efficacité comprise entre $1.10^{-2}$ et $1.10^{-3}$
$1^+$ efficacité voisine de $1.10^{-3}$
2 efficacité comprise entre $1.10^{-3}$ et $1.10^{-4}$
$2^+$ efficacité comprise entre $1.10^{-4}$
3 efficacité comprise entre $1.10^{-4}$ et $1.10^{-5}$
$3^+$ efficacité voisine de $1.10^{-5}$
4 efficacité comprise entre $1.10^{-5}$ et $1.10^{-6}$
$4^+$ efficacité voisine de $1.10^{-6}$
5 efficacité comprise entre $1.10^{-6}$ et $1.10^{-7}$

Les seuils fongistatiques des mélanges sont déterminés pour les souches suivantes :

- Coriolus versicolor,
- Coniophora Puteana,
- Pullularia Pullulans,
- Chaetomium globosum,
- Sterrigmatocystis nigra (Aspergillus niger)

Les résultats obtenus sont consignés dans le tableau suivant et donnés par comparaison avec ceux obtenus dans les mêmes conditions expérimentales avec le pentachlorophénol.

| Activité fongistatique sur | Seuils d'efficacité (classes) | |
|---|---|---|
| | composé n° 28 | PENTACHLOROPHENOL |
| Coriolus versicolor | 4 | 3 |
| Coniophora puteana | 4+ | 3 |
| Pullularia pullulans | 4 | 3 |
| Chaetomium globosum | 3 | 3 |
| Aspergillus niger | 2+ | 3 |

TABLEAU (I)

| Composé n° | $X^1$ | $R^1$ | $R^2$ | Diastéréo-isomères | Point de fusion | delta (RMN) |
|---|---|---|---|---|---|---|
| 1 (ex. 1) | H | H | $CH_3$- | A+B | huile | 5,15 5,02 |
| 2 | H | H | $C_2H_5$- | A | 88 | 5,27 |
| 3 | H | H | $C_2H_5$- | B | huile | 5,14 |
| 4 | H | H | n-$C_3H_7$- | A | huile | 5,26 |
| 5 | H | H | n-$C_3H_7$- | B | huile | 5,12 |
| 6 | H | H | n-$C_4H_9$- | A+B | huile | 5,25 5,11 |
| 7 | H | H | iso-$C_3H_7$- | A | huile | 5,39 |
| 8 | H | H | iso-$C_3H_7$- | B | 70 | 5,24 |
| 9 | Cl | H | $CH_3$- | A+B | huile | 5,20 5,04 |
| 10 (ex. 2) | Cl | H | $C_2H_5$- | A | 61 | 5,30 |
| 11 (ex. 2) | Cl | H | $C_2H_5$- | B | 164 | 5,14 |
| 12 | Cl | H | n-$C_3H_7$- | A+B | huile | 5,14 5,31 |
| 13 | Cl | H | n-$C_3H_7$- | A | huile | 5,31 |
| 14 | Cl | H | n-$C_3H_7$- | B | 116 | 5,14 |

TABLEAU I (suite)

| Composé n° | $X^1$ | $R^1$ | $R^2$ | Diastéréo-isomères | Point de fusion | delta (RMN) |
|---|---|---|---|---|---|---|
| 15 | Cl | H | iso-$C_3H_7$- | A | 100 | 5,42 |
| 16 | Cl | H | iso-$C_3H_7$- | B | 149 | 5,26 |
| 17 | Cl | H | n-$C_4H_9$- | A (85 %) | huile | 5,30 |
| 18 | Cl | H | n-$C_4H_9$- | B (90 %) | huile | 5,14 |
| 19 | Cl | H | n-$C_5H_{11}$- | A (60 %) | huile | 5,30 |
| 20 | Cl | H | n-$C_5H_{11}$- | B (90 %) | huile | 5,13 |
| 21 | Cl | H | Cl-$CH_2$-$CH_2$- | A | 103 | 5,35 |
| 22 | Cl | H | Cl-$CH_2$-$CH_2$- | B | 135 | 5,19 |
| 23 | Cl | H | cyclohexyl | A+B | huile | 5,47<br>5,31 |
| 24 | Cl | H | cyclohexyl | B | 118 | 5,31 |
| 25 | Cl | H | p-Cl-$C_6H_4$-O-$CH_2$-$CH_2$- | A | huile | 5,39 |
| 26 | Cl | H | p-Cl-$C_6H_4$-O-$CH_2$-$CH_2$- | B | 128 | 5,22 |
| 27 | Cl | $CH_3$ | n-$C_4H_9$- | A+B+C+D | huile | |
| 28 | Cl | $CH_3$ | $CH_3$- | A+B+C+D | huile | 4,90/4,74<br>4,74/4,55 |
| 29 | Cl | $CH_3$ | $C_2H_5$- | A+B+C+D | huile | 5,01/4,84<br>4,82/4,62 |
| 30 | Cl | $CH_3$ | n-$C_3H_7$- | A+B+C+D | huile | 5,01/4,83<br>4,81/4,60 |

TABLEAU I (suite)

| Composé n° | $X^1$ | $R^1$ | $R^2$ | Diastéréo-isomères présents | Point de fusion en °C | delta (RMN) |
|---|---|---|---|---|---|---|
| 31 | Cl | $n-C_3H_7-$ | $CH_3-$ | A+B+C+D | huile | |
| 32 | Cl | $n-C_3H_7-$ | $C_2H_5-$ | A+B+C+D | huile | |
| 33 | Cl | H | $iso-C_4H_9-$ | A | 71° | 5,29 |
| 34 | Cl | H | $iso-C_4H_9-$ | B | 80° | 5,12 |
| 35 | Cl | $CH_3$ | $n-C_3H^7$ | A+B | huile | 5,00/4,81 |
| 36 | Cl | $n-C_3H_7-$ | $C_2H_5-$ | C+D | huile | 4,83/4,61 |
| 37 | Cl | H | allyle | A | 75°C | |
| 38 | Cl | H | allyle | B | 181°C | |
| 39 | Cl | $CH_3$ | $F-CH_2-CH_2-$ | A+B | huile | 5,05/4,90 |
| 40 | Cl | $CH_3$ | $F-CH_2-CH_2-$ | C+D | huile | 4,91/4,66 |
| 41 | Cl | $CH_3$ | $F-CH_2-CH_2-$ | A+B+C+D | huile | 5,05/4,91<br>4,90/4,66 |
| 42 | Cl | $CH_3$ | $CF_3-CH_2-$ | A+B | huile | 5,09/4,95 |
| 43 | Cl | H | $F-CH_2-CH_2-$ | A | 107° | 5,29 |
| 44 | Cl | H | $F-CH_2-CH_2-$ | B | 158° | 5,14 |
| 45 | Cl | H | $C_2H_5-$ | A+B | 57° | 5,30/5,14 |

TABLEAU I (suite)

| Composé n° | $X^1$ | $R^1$ | $R^2$ | Diastéréo-isomères présents | Point de fusion en °C | delta (RMN) |
|---|---|---|---|---|---|---|
| 47 | Cl | H | H | A+B | 171° | |
| 48 | Cl | n-$C_3H_7$ | H | A+B+C+D | 152° | |
| 49 | F | H | $C_2H_5$- | A+B | huile | 5,24<br>5,08 |
| 50 | F | H | $C_2H_5$- | A+B | huile | 5,24<br>5,12 |

TABLEAU (II)

Doses minimales inhibitrices en ppm

| Composé n° | CERC | HELM G | PYRE | HELM T | SEPT N | FUS ROS | FUS NIV | FUS CULM | RHIZ C |
|---|---|---|---|---|---|---|---|---|---|
| 1 | | 100 | 100 | 100 | 100 | | | 100 | |
| 2 | | 33 | | | 33 | | | 33 | |
| 3 | | 100 | | | | | | | |
| 4 | 100 | 100 | 100 | 100 | 100 | 100 | 33 | 33 | 100 |
| 5 | 100 | | | | | | 100 | 100 | 100 |
| 6 | 100 | 100 | 100 | 100 | 100 | 100 | 33 | 33 | 100 |
| 7 | 100 | 100 | 100 | 100 | 100 | 100 | 33 | 33 | 100 |
| 8 | 11 | 100 | | | | 33 | 33 | 33 | 100 |
| 9 | 1,1 | 11 | 3,3 | 3,3 | 3,3 | 33 | 33 | 11 | 11 |
| 10 | 3,3 | 11 | 3,3 | 1,1 | 1,1 | 11 | 11 | 11 | 1,1 |
| 11 | 3,3 | 100 | 33 | 33 | 1,1 | 100 | 100 | 33 | 33 |
| 12 | 1,1 | 33 | 11 | 1,1 | 3,3 | 33 | 33 | 33 | 11 |
| 15 | 100 | 33 | 33 | 33 | 33 | 100 | 100 | 33 | 33 |
| 16 | 11 | | | | 100 | 33 | 33 | 33 | 100 |
| 17 | 1,1 | | 11 | 3,3 | 3,3 | 33 | 33 | 33 | 11 |
| 18 | 3,3 | | 33 | 33 | 33 | 33 | 33 | 33 | 11 |
| 19 | 3,3 | | 11 | 11 | 11 | 33 | 33 | 33 | 11 |
| 20 | 1,1 | 100 | 33 | 33 | 100 | 33 | 100 | 33 | 11 |
| 21 | 100 | 11 | 11 | 11 | 11 | 100 | 100 | 100 | 33 |
| 22 | 100 | 100 | 100 | 100 | 100 | 100 | 100 | 100 | 100 |
| 25 | | 100 | 100 | 100 | | | | | |

TABLEAU (III)
Dose minimales inhibitrices en ppm

| Champignon | Composé n° | dose minimale inhibitrice |
|---|---|---|
| ALT | 29 | 30 |
| ASP | 29 | 100 |
| BOT résistant à l'iprodione | 29 | 10 |
| CBET | 29 | 100 |
| CARA | 29 | 3 |
| CLAD | 29 | 30 |
| FUS CULM | 29 | 30 |
| FUS OX | 29 | 100 |
| HELM OR | 29 | 100 |
| PEN | 29 | 100 |
| PES | 29 | 30 |
| PHI | 29 | 30 |
| PHB | 29 | 10 |
| PHF | 29 | 100 |
| PHL | 29 | 10 |
| PHY CI | 29 | 100 |
| PHY CC | 29 | 100 |
| PHY CP | 29 | 100 |
| PHY IN | 29 | 100 |
| PHY PA | 29 | 100 |
| PHY ME | 29 | 100 |
| PHY SY | 29 | 100 |
| SCL S | 29 | 10 |
| UST | 29 | 3 |

TABLEAU (III) suite

| | | |
|---|---|---|
| VERT | 29 | 10 |
| ASCO | 29 | 30 |
| GUIG | 29 | 100 |
| CRO | 29 | 10 |
| SEPT T | 29 | 100 |
| PHV | 29 | 30 |
| SCL M | 29 | 10 |
| CORY | 29 | 30 |
| VENT | 31 | 10 |
| | 28 | 3 |
| | 29 | 3 |
| | 35 | 10 |
| WHE | 29 | 10 |
| | 31 | 30 |
| | 28 | 10 |
| | 35 | 10 |
| PIR | 28 | 10 |
| | 29 | 10 |
| | 35 | 30 |
| MON | 31 | 10 |
| | 28 | 10 |
| | 29 | 1 |
| | 35 | 10 |

TABLEAU (III) (suite)

| | | |
|---|---|---|
| MSPH | 28 | 30 |
| | 29 | 1 |
| MARS | 28 | 10 |
| | 29 | 10 |

TABLEAU (IV)

Dose minimale inhibitrice en ppm

| Composé n° / champignon | 28 | 29 | 30 | 31 | 32 | 33 | 34 | 35 | 36 | 37 |
|---|---|---|---|---|---|---|---|---|---|---|
| BOT | 10 | 10 | 30 | 100 | | | | 10 | | 10 |
| FUS OX | 100 | 100 | 100 | > 100 | > 100 | > 100 | 100 | > 100 | > 100 | > 100 |
| PYT | 100 | > 100 | > 100 | > 100 | > 100 | 100 | 100 | | > 100 | > 100 |
| RHIZ S | 30 | 100 | 100 | 100 | 30 | 30 | 100 | 30 | 100 | 3 |
| FUS CULM | 30 | 30 | 30 | 100 | 100 | 100 | 100 | 30 | 100 | 100 |
| FUS NIV | 30 | 30 | 30 | 100 | 100 | 100 | 100 | 100 | 100 | 100 |
| FUS ROS | 30 | 100 | 100 | 100 | 100 | 100 | 100 | 30 | 30 | 100 |
| CERC | 10 | ≤ 1 | 10 | 100 | 30 | 30 | 100 | 10 | 30 | 10 |
| HELM G | 10 | 3 | 10 | 30 | 100 | 100 | 100 | 10 | 100 | 30 |
| PYRE | 10 | 10 | 10 | 100 | 100 | 100 | 100 | 10 | 100 | 10 |
| HELM T | 3 | 3 | 3 | 30 | 100 | 100 | 100 | 10 | 100 | 10 |
| SEPT N | 3 | ≤ 1 | 3 | 30 | 100 | 30 | 100 | 10 | 100 | 10 |
| RHIZ C | 30 | ≤ 1 | 10 | 100 | 100 | 100 | 100 | 10 | 10 | 100 |
| SEPT T | > 100 | | 100 | > 100 | | > 100 | | | | 100 |

TABLEAU (IV) (suite)
Dose minimale inhibitrice en ppm

| Composé n° / champignon | 38 | 39 | 40 | 41 | 42 | 43 | 44 | 49 | 50 |
|---|---|---|---|---|---|---|---|---|---|
| BOT | | 10 | | 10 | 30 | 30 | | 100 | 100 |
| FUS OX | > 100 | >100 | > 100 | >100 | >100 | | | | |
| PYT | > 100 | | >100 | | | >100 | | | |
| RHIZ S | 30 | 100 | 100 | 100 | 100 | 100 | | | 100 |
| FUS CULM | 100 | 30 | 100 | 30 | 100 | 100 | 100 | 100 | 100 |
| FUS NIV | 100 | 30 | 100 | 100 | 100 | 100 | >100 | 100 | 100 |
| FUS ROS | 100 | 100 | 100 | 100 | 100 | 100 | | >100 | 100 |
| CERC | 10 | 10 | 100 | 10 | 100 | 3 | 100 | 10 | 10 |
| HELM G | 100 | 100 | 100 | 30 | 100 | 30 | > 100 | 100 | 100 |
| PYRE | 100 | 10 | 100 | 10 | 10 | 30 | >100 | 100 | 10 |
| HELM T | 100 | 10 | 100 | 10 | 10 | 30 | 100 | 30 | 10 |
| SEPT N | 100 | 10 | 100 | 10 | 10 | 30 | 100 | 30 | 30 |
| RHIZ C | 100 | 30 | 100 | 30 | 30 | 100 | >100 | 100 | 100 |
| SEPT T | 100 | | | | | > 100 | > 100 | | |

0151084

TABLEAU (V)

Dose minimale inhibitrice en ppm

| Composé n° / champignon | 28 | 29 | 30 | 31 | 32 | 33 | 34 | 35 | 36 | 37 |
|---|---|---|---|---|---|---|---|---|---|---|
| BOT | 1000 | 330 | | | | | | 1000 | | 1000 |
| ERYG | 110 | 110 | 110 | 330 | 110 | 110 | 330 | 110 | 110 | 1000 |
| PUCR | 110 | 110 | 110 | 330 | 110 | 110 | >1000 | 110 | >1000 | 1000 |
| PLA | 100 | | | 1000 | 330 | 1000 | 1000 | >1000 | >1000 | >1000 |
| PIR | 1000 | 1000 | | 1000 | | >1000 | | 110 | 1000 | |
| CBET | 1000 | 110 | | 1000 | 330 | 1000 | 1000 | 330 | 1000 | 1000 |
| PUCS | $\leq$ 250 | $\leq$ 250 | $\leq$ 250 | | | | | | | |
| ERYC | 125 | 125 | | | | | | | | |

TABLEAU (V) (suite)

Dose minimale inhibitrice en ppm

| Composé n° / champignon | 38 | 39 | 40 | 41 | 42 | 43 | 44 | 49 | 50 |
|---|---|---|---|---|---|---|---|---|---|
| BOT | | >1000 | | | >1000 | 1000 | | 1000 | |
| ERYG | 1000 | 330 | 1000 | 1000 | 110 | 330 | >1000 | 330 | 1000 |
| PUCR | | 1000 | >1000 | 1000 | 1000 | 1000 | | >1000 | >1000 |
| PLA | | >1000 | >1000 | | >1000 | | | | >1000 |
| PIR | | | | | | | | | |
| CBET | >1000 | 330 | 1000 | 330 | 330 | 330 | 1000 | >1000 | |
| PUCS | | | | | | | | | |
| ERYC | | | | | | | | | |

TABLEAU (VI)
Dose minimale inhibitrice en ppm

Les chiffres entre parenthèses indiquent le taux d'inhibition en pourcentage à la dose mentionnée

| Composé n° / champignon | 45 | 29 | 12 | 35 | 9 | 28 | 47 | 48 |
|---|---|---|---|---|---|---|---|---|
| BOT | 30 | 10 | 100 | 10 | 30 | 10 | 100 (0) | 100 |
| FUS CULM | 100 | 30 | 100 | 30 | 11 | 30 | 100 | >100 (80) |
| FUS NIV | 100 | 30 | 100 | 100 | 33 | 30 | >100 | 30 |
| FUS ROS | 100 | 100 | >100 | 30 | 33 | 30 | >100 (80) | >100 (50) |
| CERC | 30 | ≤ 1 | 30 | 10 | 10 | 10 | 10 | 10 |
| HELM G | 30 | 10 | 100 | 10 | 11 | 10 | >100 | 30 |
| PYRE | 10 | 3 | 30 | 10 | 3,3 | 10 | >100 | 30 |
| HELM T | 3 | 3 | 10 | 10 | 3,3 | 3 | 100 | 10 |
| SEPT N | 3 | ≤ 1 | 30 | 10 | 3,3 | 3 | 100 | 10 |
| RHIZ C | 10 | ≤ 1 | 30 | 10 | 11 | 30 | >100 | 30 |

TABLEAU (VII)

| Champignon | Composé n° | dose en g/ha | pourcentage d'épis malades | % de surface foliaire malade | Modalités d'observations des résultats |
|---|---|---|---|---|---|
| PUCS | (témoin) | 0 | | 40 | 23 jours après trai- |
| | | 250 | | 17,5 | tement ; observation |
| | 45 | 500 | | 16,3 | sur toute la plante |
| | | 250 | | 7,9 | malade, au stade 4 |
| | 29 | 500 | | 6,3 | feuilles |
| PUCS | (témoin) | 0 | | 82,3 | 14 jours après trai- |
| | 45 | 125 | | 3,4 | tement ; observation |
| | 29 | 125 | | 1,2 | sur la 3ème feuille |
| PUCS | (témoin) | 0 | | 25,9 | 15 jours après 1er |
| | 45 | 125 | | 6,7 | traitement, observation |
| | 29 | 125 | | 3,5 | sur la 1ère feuille |
| ERYG | (témoin) | 0 | | 25,9 | 14 jours après trai- |
| | 45 | 250 | | 4,7 | tement ; observation |
| | 29 | 250 | | 1,0 | sur la 3ème feuille |
| PUCS | (témoin) | 0 | 100 | 47,7 | 22 jours après 2ème |
| | 12 | 125 | 88 | 5,8 | traitement pour les |
| | 28 | 125 | 64 | 0,8 | épis ; 7 jours après |
| | | | | | 2ème traitement pour |
| | | | | | le contrôle sur la |
| | | | | | 2ème feuille |
| ERYG | (témoin) | 0 | | 10,3 | 20 jours après 2ème |
| | 12 | 125 | | 0,9 | traitement, contrôle |
| | 28 | 125 | | 0,1 | sur la 1ère feuille |
| PUCS | (témoin) | 0 | 96 | | 24 jours après le |
| | 45 | 125 | 74 | | traitement |
| | | 250 | 74 | | |
| | 29 | 125 | 54 | | |
| | | 250 | 46 | | |
| | 12 | 125 | 91 | | |
| | | 250 | 87 | | |
| | 28 | 125 | 39 | | |
| | | 250 | 46 | | |
| | | | pourcentage de tiges malades | | |
| CERC | (témoin) | 0 | 93 | | |
| | 45 | 500 | 96 | | |
| | | 1000 | 90 | | |
| | 29 | 500 | 82 | | |
| | | 1000 | 72 | | 56 jours après |
| | 12 | 500 | 93 | | traitement ; |
| | | 1000 | 85 | | contrôle sur la |
| | 28 | 500 | 82 | | tige principale |
| | | 1000 | 63 | | |

REVENDICATIONS

1) Composés caractérisés en ce qu'ils ont pour formule

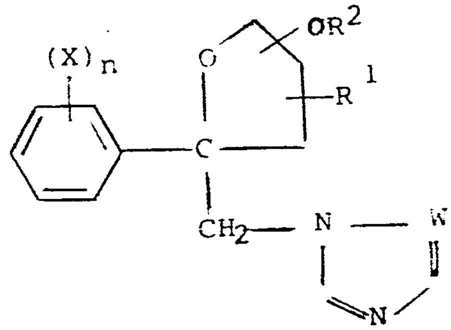

(I)

dans laquelle :

X est un atome d'halogène ou un groupe cyano ou nitro, ou un groupe alkyle ou alkoxy ,éventuellement halogéné,

n est un nombre entier, positif ou nul, inférieur à 6,

W représente un groupe trivalent constitué soit d'un groupe =CH- soit d'un atome d'azote =N-

$R^1$ représente l'atome d'hydrogène ou un radical alkyle, $R^2$ représente un atome d'hydrogène ou un radical hydrocarboné éventuellement substitué.

2) Composés selon la revendication 1 caractérisés en ce que W est l'atome d'azote et que $R^2$ est un radical alkyle, cycloalkyle, aryle ou aralkyle éventuellement substitué par des atomes d'halogène ou des groupes alkoxy ou aryloxy.

3) Composés selon l'une des revendications 1 ou 2 caractérisés en ce que n est égal à 2.

4) Composés selon l'une des revendications 1 à 3 caractérisés en ce que X est le fluor, le brome ou le chlore, ou un radical alkyle ou alkoxy ayant de 1 à 4 atomes de carbone, ou $CF_3$.

5) Composés, caractérisés en ce qu'ils ont pour formule

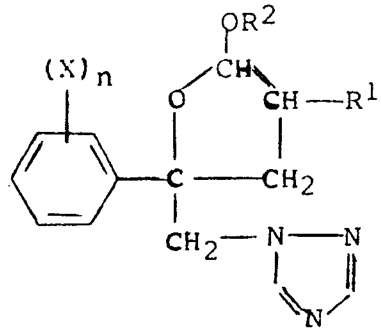

(Ia)

dans laquelle $R^1$, $R^2$, X et n ont l'une des significations indiquées dans l'une des revendications 1 à 4.

6) Composés, éventuellement utilisables à titres d'intermédiaires pour la préparation de composés selon l'une des revendications 1 à 5 caractérisés en ce qu'ils ont pour formule

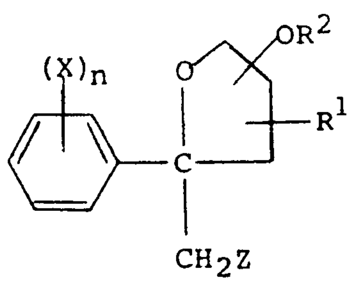

(II)

dans laquelle Z est un atome de chlore ou de brome et X, n, $R^1$ et $R^2$ ont l'une des significations données dans l'une des revendications 1 à 5.

7) Composés selon la revendication 6 caractérisés en ce qu'ils ont pour formule

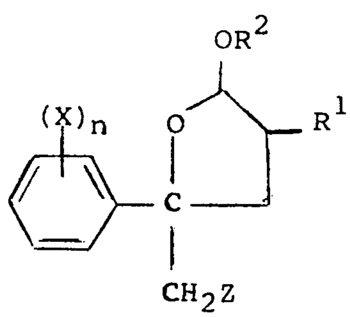

(IIa)

où les divers symboles ont même signification que dans la revendication 6.

8) Composés, éventuellement utilisables à titre

d'intermédiaires pour la préparation de composés selon l'une des revendications 1 à 7 caractérisés en ce qu'il ont pour formule

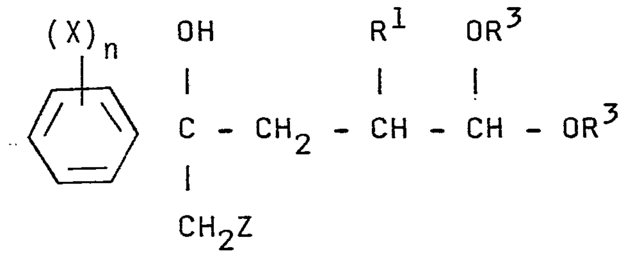

(III)

dans laquelle $R^1$, X, Z et n ont l'une des significations indiquées dans l'une des revendications 1 à 6, et $R^3$ est un radical organique ou deux radicaux $R^3$ forment ensemble un radical unique organique divalent.

9) Composés selon la revendication 8 caractérisés en ce que $R^3$ est un radical alkyle ou que les deux radicaux $R^3$ forment ensemble un radical unique alkylène.

10) Composés, éventuellement utilisables à titre d'intermédiaires pour la préparation de composés selon l'une des revendications 1 à 5 caractérisés en ce qu'ils ont pour formule

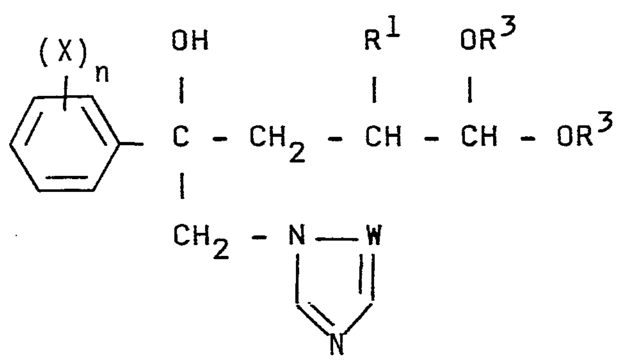

(IV)

les divers symboles W ,$R^1$, $R^3$, X et n ayant l'une des significations indiquées dans l'une des revendications 1 à 5.

11) Composés, éventuellement utilisables à titre d'intermédiaires pour la préparation de composés

selon l'une des revendications 1 à 5 et 10 caractérisés en ce qu'ils ont pour formule

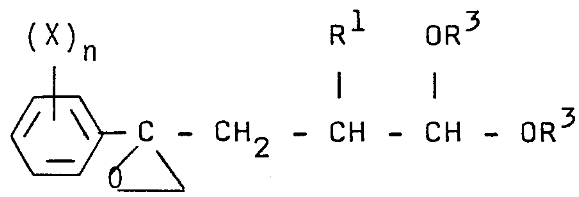

(V)

les divers symboles $R^1$, $R^3$, X et n ayant l'une des significations indiquées dans l'une des revendications 1 à 5 et 8 ou 9.

12) Procédé de préparation de composés selon l'une des revendications 1 à 5 caractérisé en ce qu'on fait réagir un composé de formule

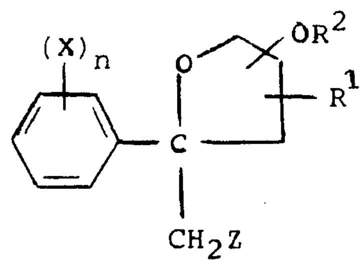

(II)

dans laquelle Z est un atome de chlore ou de brome, et X, n, $R^1$ et $R^2$ ont l'une des significations indiquées dans l'une des revendications 1 à 5, avec un dérivé alcalin d'un imidazole ou triazole.

13) Procédé selon la revendication 12 caractérisé en ce que la température est comprise entre 50 et 250°C, et/ou que le milieu contient un solvant polaire aprotique, et/ou que la concentration globale en réactifs est comprise entre 1 et 50 %.

14) Procédé de préparation de composés selon la revendication 7 caractérisé en ce qu'on fait réagir, en présence d'un catalyseur acide, un alcool de formule $R^2OH$ avec un composé selon l'une des revendications 8 ou 9.

15) Procédé selon la revendication 14 caractérisé en ce que le catalyseur est un acide protique fort ou un acide de Lewis et que la température est comprise dans la zone allant de 50° à la température d'ébullition du milieu réactionnel.

16) Procédé de préparation de composés selon la revendication 8 caractérisé en ce qu'on fait réagir un composé carbonylé de formule

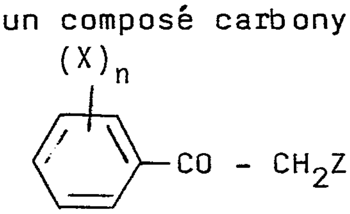 (IIIa)

avec le dérivé organomagnésien préparé à partir du composé de formule

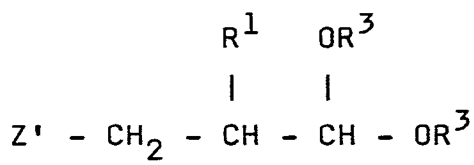 (IIIb)

symboles ayant les significations indiquées dans les revendications 8 ou 9.

17) Procédé de préparation de composés selon l'une des revendications 1 à 5 caractérisé en ce qu'on fait agir un alcool $R^2OH$ en présence d'un catalyseur acide avec un composé selon la revendication 10.

18) Compositions fongicides, caractérisées en ce qu'elles contiennent comme matière active un produit selon l'une des revendications 1 à 5, cette matière active étant en association avec au moins un support inerte, acceptable en agriculture.

19) Compositions selon la revendication 18, caractérisées en ce qu'elles contiennent 0,5 à 95 % de matière active.

20) Compositions selon la revendication 19, caractérisées en ce qu'elles contiennent 1 à 95 %

de support et 0,1 à 20 % d'agent tensioactif.

21) Procédé pour lutter contre les maladies fongiques des cultures, caractérisé en ce qu'on applique une dose efficace d'une matière active selon l'une des revendications 1 à 5.

22) Procédé selon la revendication 21, caractérisé en ce qu'on applique une composition selon l'une des revendications 18 à 20, la matière active étant appliquée à raison de 0,05 à 5 kg/ha, de préférence de 0,02 à 2 kg/ha.

23) Procédé selon l'une des revendications 21 ou 22, caractérisé en ce que la culture traitée est une céréale et/ou que la culture est atteinte ou susceptible d'être atteinte par le piétin-verse.

24) Composés selon la revendication 1 caractérisés en ce que $R^2$ est un radical alkyle substitué par un atome d'halogène, de préférence le fluor ou le chlore.

25) Composés selon la revendication 24 caractérisés en ce que n est égal à 2 et W est l'atome d'azote.

26) Composés caractérisés en ce qu'ils ont pour formule:

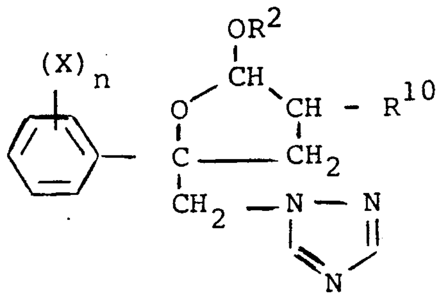

dans laquelle
- $R^2$, X et n ont l'une des significations indiquées dans les revendications 1 à 4,
- $R^{10}$ est un radical alkyle, de préférence un radical alkyle inférieur.

27) Composés selon la revendication 26 caractérisés en ce que n est égal à 2 et/ou $R^{10}$ est le radical

méthyle et que, de préférence, X est l'atome de chlore.

28) Composés, éventuellement utilisables à titres d'intermédiaires pour la préparation de composés selon l'une des revendications 24 à 27 caractérisés en ce qu'ils ont pour formule

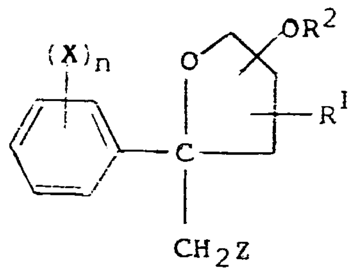

(II)

dans laquelle Z est un atome de chlore ou de brome et X, n, $R^1$ et $R^2$ ont l'une des significations données dans l'une des revendications 24 à 27, et $R^1$ peut représenter $R^{10}$, $R^{10}$ ayant la signification donnée dans la revendication 26.

29) Composés selon la revendication 28 caractérisés en ce qu'ils ont pour formule

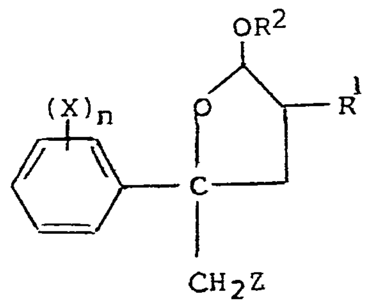

(IIa)

où les divers symboles ont même signification que dans la revendication 28.

30) Composés, éventuellement utilisables à titre d'intermédiaires pour la préparation de composés selon l'une des revendications 26 à 27 caractérisés en ce qu'il ont pour formule

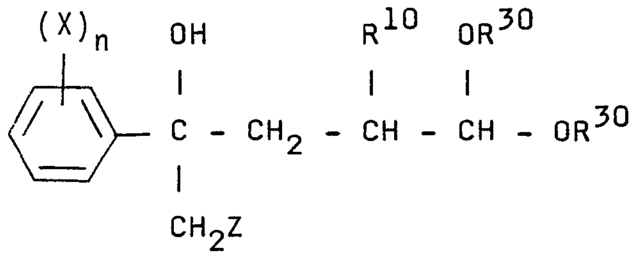

(III)

dans laquelle $R^{10}$, X, Z et n ont l'une des significations indiquées dans l'une des revendications 26 à 27 et les deux radicaux $R^{30}$, identiques ou différents, représentent un radical organique, de préférence alkyle inférieur .

31) Composés, éventuellement utilisables à titre d'intermédiaires pour la préparation de composés selon l'une des revendications 24 à 27 caractérisés en ce qu'ils ont pour formule

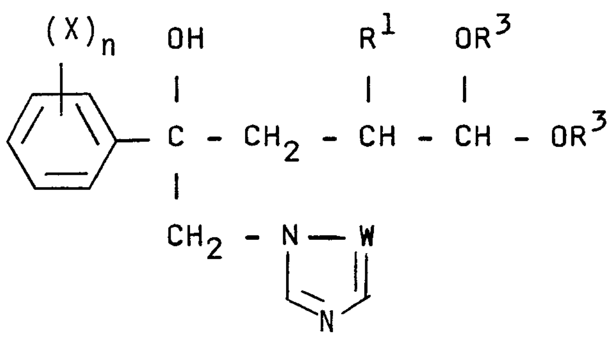

(IV)

les divers symboles W, $R^1$, X et n ayant l'une des significations indiquées dans l'une des revendications 24 à 27, et le radical $R^3$ représentant un radical organique, de préférence un radical alkyle, les deux radicaux $R^3$ pouvant en outre, lorsque $R^1$ est l'atome d'hydrogène, constituer ensemble un radical unique divalent, de préférence un radical alkylène.

32) Composés, éventuellement utilisables à titre d'intermédiaires pour la préparation de composés selon l'une des revendications 24 à 27 et 30 caractérisés en ce qu'ils ont pour formule

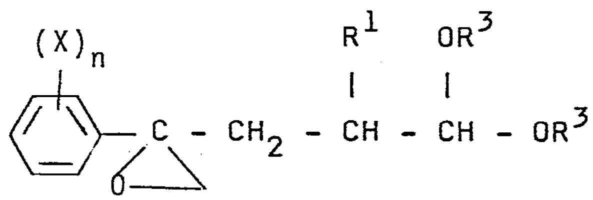

(V)

les divers symboles $R^1$, $R^3$, X et n ayant l'une des significations indiquées dans l'une des revendications 24 à 27 et 30.

33) Procédé de préparation de composés selon l'une des revendications 24 à 27 caractérisé en ce qu'on fait réagir un composé de formule

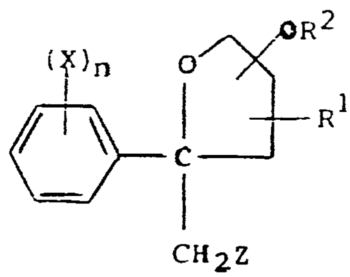

(II)

dans laquelle Z est un atome de chlore ou de brome, et X, n, $R^1$ et $R^2$ ont l'une des significations indiquées dans l'une des revendications 24 à 27 avec un dérivé alcalin d'un imidazole ou triazole.

34) Procédé selon la revendication 33 caractérisé en ce que la température est comprise entre 50 et 250°C en milieu solvant polaire aprotique, la concentration globale en réactifs étant comprise entre 1 et 50 %.

35) Procédé de préparation de composés selon la revendication 29 caractérisé en ce qu'on fait réagir, en présence d'un catalyseur acide, un alcool de formule $R^2OH$ avec un composé selon la revendication 30.

36) Procédé selon la revendication 35 caractérisé en ce que le catalyseur est un acide protique fort ou un acide de Lewis et que la température est comprise

dans la zone allant de 50° à la température d'ébullition du milieu réactionnel.

37) Procédé de préparation de composés selon la revendication 30 caractérisé en ce qu'on fait réagir un composé carbonylé de formule

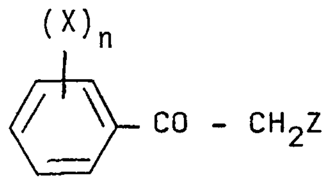

(IIIa)

avec le dérivé organomagnésien préparé à partir du composé de formule

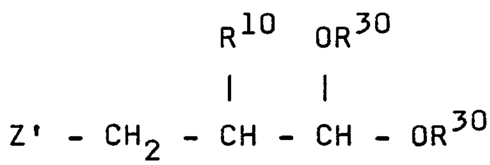

(IIIb)

symboles ayant les significations indiquées dans la revendication 30.

38) Procédé de préparation de composés selon l'une des revendications 24 à 27 caractérisé en ce qu'on fait agir un alcool $R^2OH$ en présence d'un catalyseur acide avec un composé selon la revendication 31.

39) Compositions fongicides, caractérisées en ce qu'elles contiennent comme matière active un produit selon l'une des revendications 24 à 27, cette matière active étant en association avec au moins un support inerte, acceptable en agriculture.

40) Compositions selon la revendication 39, caractérisées en ce qu'elles contiennent 0,5 à 95 % de matière active.

41) Compositions selon la revendication 40, caractérisées en ce qu'elles contiennent 1 à 95 % de support et 0,1 à 20 % d'agent tensioactif.

42) Procédé pour lutter contre les maladies fongiques des cultures, caractérisé en ce qu'on applique une

dose efficace d'une matière active selon l'une des revendications 24 à 27.

43) Procédé selon la revendication 42, caractérisé en ce qu'on applique une composition selon l'une des revendications 39 à 41, la matière active étant appliquée à raison de 0,05 à 5 kg/ha, de préférence de 0,02 à 2 kg/ha.

44) Procédé selon l'une des revendications 42 ou 43, caractérisé en ce que la culture traitée est une céréale.

45) Procédé pour lutter contre les atteintes fongiques des cultures caractérisé en ce qu'on applique une dose efficace d'une matière active selon l'une des revendications 1 à 5 et 24 à 27 à une culture atteinte ou susceptible d'être atteinte par un ou plusieurs champignons choisis dans le groupe constitué par (noms latins) Pseudocercosporella herpotrichoïdes, Helminthosporium gramineum, Pyrenophorae avenae, Septoria nodorum, Helminthosporium teres, Fusarium roseum, Fusarium nivale, Fusarium culmorum, Rhizoctonia cerealis.

46) Procédé pour lutter contre les atteintes fongiques des cultures caractérisé en ce qu'on applique une dose efficace d'une matière active selon l'une des revendications 1 à 5 et 24 à 27 à une culture atteinte ou susceptible d'être atteinte par un ou plusieurs champignons choisis dans le groupe constitué par (noms latins)

Cercospora beticola
Peronospora tabacina
Erysiphe cichoracearum
Pythium spp
Pyrenophora avenae
Whetzelinia sclerotiorum
Monilia laxa
Mycosphaerella fijiensis

Marssonina panattoniana
Alternaria solani
Aspergillus niger
Cladosporium herbarum
Penicillium expansum
Pestalozzia sp
Phialophora cinerescens
Phoma betae
Phoma foveata
Phoma lingam
Verticillium dahliae
Ascochyta pisi
Guignardia bidwellii
Corticium rolfsii
Phomopsis viticola
Sclerotinia sclerotiorum
Sclerotinia minor
Phytophthora cinnamomi
Phytophthora cactorum
Phytophthora capsici
Phytophthora parasitica
Phytophthora megasperma
Phytophthora syringae
Coryneum cardinale

47) Procédé pour lutter contre les atteintes fongiques des produits lignocellulosiques et notamment du bois caractérisé en ce qu'on applique une dose efficace d'une matière active selon l'une des revendications 1 à 5 et 24 à 27 à un produit lignocellulosique ou susceptible d'être atteinte par un ou plusieurs champignons choisis dans le groupe constitué par ceux des genres Pullularia, Chaetomium, Aspergillus, Coniophora.